# EUROPEAN PATENT APPLICATION

(11) **EP 1 897 875 A1**
(43) Date of publication of application: **12.03.2008**
(21) Application number: 07017137.6
(22) Date of filing: 31.08.2007
(51) Int. Cl.: C07D 401/04

(54) **Process for producing 1-pyridin-4-YL-indoles**

(30) Priority: 07.09.2006 JP 2006242875
(71) Applicant: Fujifilm Finechemicals Co. Ltd., Chuo-ku Tokyo 104-0031 (JP)
(72) Inventor: Aoki, Hidenori, Hiratsuka-shi Kanagawa (JP); Tsujiyama, Shinichiro, Hiratsuka-shi Kanagawa (JP); Ishikawa, Yuji, Hiratsuka-shi Kanagawa (JP); Harada, Susumu, Hiratsuka-shi Kanagawa (JP)
(74) Representative: Grünecker, Kinkeldey, Stockmair & Schwanhäusser Anwaltssozietät

(57) **Abstract**

A process for producing a 1-pyridin-4-yl-indole represented by formula (III) as defined in the specification, which comprises reacting a pyridine compound represented by formula (I) as defined in the specification with an indole compound represented by formula (II) as defined in the specification in the presence of a base at 50 to 200°C.

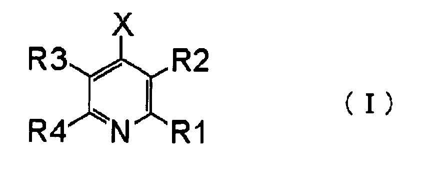

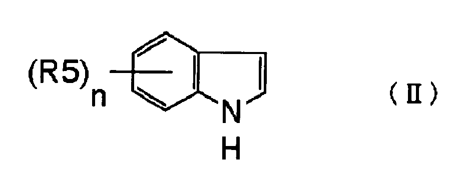

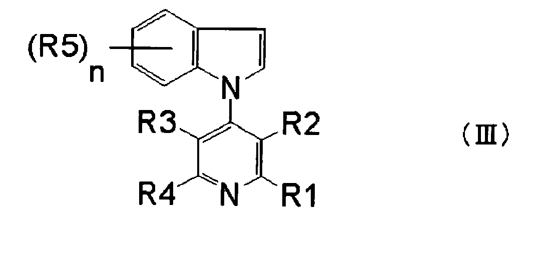

## Description

### Background of the Invention

### 1. Field of the Invention

The present invention relates to a process for producing 1-pyridin-4-yl-indoles useful in the fields of medicines, agricultural chemicals, organic electroluminescence elements, catalyst ligands, solar cell elements and the like.

### 2. Description of the Related Art

1-Pyridin-4-yl-indoles are useful intermediates for medicines, agricultural chemicals, organic electroluminescence elements, catalyst ligands, solar cell elements and the like and, particularly in the field of medicines, are extremely useful as intermediates for medicines such as psychotic depression, cardiovascular diseases and inflammatory diseases.

As processes for synthesizing 1-pyridin-4-yl-indoles, processes using a heavy metal catalyst such as a process of Ullmann type reaction using a combination of copper powder or a copper salt and various copper ligands (see WO2003/104222) and a process of C-N bond-forming reaction using a palladium catalyst and a phosphine series ligand (see US-A-2005054631). These processes, however, involve such problems as that oxygen must be completely removed from the reaction system because the catalyst is unstable in the air, that an expensive catalyst is required, and that there are fears of contamination of products with the heavy metal used and detrimental influences of the heavy metal on the environment, thus being difficultly said to be an industrially adequate technique.

A process of performing the reaction by using only a base and not using any heavy metal catalyst has also been tried. For example, there is illustrated a process of using a 4-fluoropyridine and indole as substrates in the presence of sodium hydride (see Journal of Organic Chemistry of the USSR, 1988, vol.24, No.12, pp.2344-2351 and Heterocycles, 1994, Vol.37, No.2, pp.993-996). However, 4-fluoropyridines are expensive and difficultly available and, therefore, involve problems with respect to production cost for producing on an industrial scale.

On the other hand, processes for synthesizing 1-pyridin-2-yl-indoles by using 2-chloropyridines in the presence of sodium hydride have been disclosed (see JP-A-6-92935 and Heterocycles, 1994, Vol.37, No.2, pp.993-996). However, 2-halogenopyridines are more reactive than 4-halogenopyridines and, for example, it is reported that, when 4-chloropyridines are used for the reaction under the same conditions as in the case of using 2-chloropyridines, the reaction is extremely difficult to proceed or does not proceed at all (see Heterocycles, 1994, Vol.37, No.2, pp.993-996).

### Summary of the Invention

An object of the invention is to provide a process for producing 1-pyridin-4-yl-indoles useful in the fields of medicines, agricultural chemicals, organic electroluminescence elements, catalyst ligands, solar cell elements and the like in high yield inexpensively on an industrial scale.

As a result of intensive investigations for attaining the above-described object, the inventors have found a novel process for synthesizing 1-pyridin-4-yl-indoles which process attains the above-described subject, thus having achieved the invention. That is, the object of the invention can be attained by the following process.
(1) A process for producing a 1-pyridin-4-yl-indole represented by formula (III), which comprises reacting a pyridine compound represented by formula (I) with an indole compound represented by formula (II) in the presence of a base at 50 to 200°C: 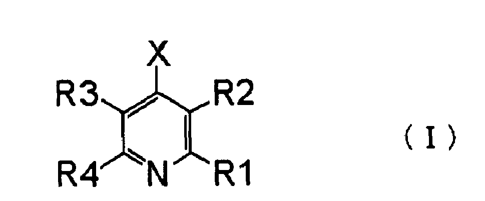 wherein X represents a chlorine atom, a bromine atom or an iodine atom;
   R1 and R4 each independently represents a hydrogen atom, an alkyl group, an alkenyl group, an alkynyl group, an aryl group, an alkoxy group, an aryloxy group, an alkylcarbonyloxy group, an arylcarbonyloxy group, a carboxy group, an alkylcarbonyl group, an arylcarbonyl group, an alkoxycarbonyl group, an aryloxycarbonyl group, a sulfo group, an alkylsulfonyl group, an arylsulfonyl group, a carbamoyl group di-substituted by at least one of alkyl and aryl, a sulfamoyl group di-substituted by at least one of alkyl and aryl, an alkylthio group, an arylthio group, an alkylthiocarbonyl group, an arylthiocarbonyl group, a nitro group, an amino group di-substituted by at least one of alkyl and aryl, a cyano group or a hetero ring residue;
   R2 and R3 each independently represents a hydrogen atom, an alkyl group, an alkenyl group, an alkynyl group, an aryl group, an alkoxy group, an aryloxy group, an alkylcarbonyloxy group, an arylcarbonyloxy group, a carboxy group, an alkylcarbonyl group, an arylcarbonyl group, an alkoxycarbonyl group, an aryloxycarbonyl group, a sulfo group, an alkylsulfonyl group, an arylsulfonyl group, a carbamoyl group di-substituted by at least one of alkyl and aryl, a sulfamoyl group di-substituted by at least one of alkyl and aryl, an alkylthio group, an arylthio group, an alkylthiocarbonyl group, an arylthiocarbonyl group, a nitro group, an amino group di-substituted by at least one of alkyl and aryl, a cyano group, a chlorine atom, a bromine atom, an iodine atom or a hetero ring residue, with R1 and R2, or R3 and R4, being optionally connected to each other to form a ring; 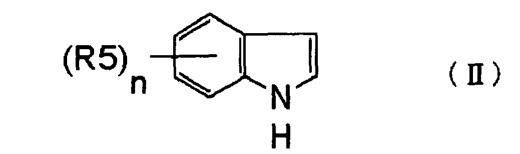 wherein R5 represents an alkyl group, an alkenyl group, an alkynyl group, an aryl group, an alkoxy group, an aryloxy group, an alkylcarbonyloxy group, an arylcarbonyloxy group, a carboxy group, an alkylcarbonyl group, an arylcarbonyl group, an alkoxycarbonyl group, an aryloxycarbonyl group, a sulfo group, an alkylsulfonyl group, an arylsulfonyl group, a carbamoyl group di-substituted by at least one of alkyl and aryl, a sulfamoyl group di-substituted by at least one of alkyl and aryl, an alkylthio group, an arylthio group, an alkylthiocarbonyl group, an arylthiocarbonyl group, a nitro group, an amino group di-substituted by at least one of alkyl and aryl, a cyano group, a halogen atom or a hetero ring residue;
   n represents an integer of from 0 to 6, and when n represents 2 or more, R5's may be the same or different from each other; and 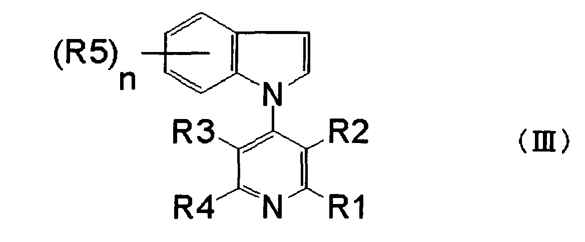 wherein R1 to R5 and n are the same as defined above.
(2) The process for producing a 1-pyridin-4-yl-indole as described in (1) above,
   wherein pKa of the base used is 15 or more.
(3) The process for producing a 1-pyridin-4-yl-indole as described in (1) or (2) above,
   wherein the base used is sodium hydride, sodium hydroxide, potassium hydroxide, sodium methoxide, sodium tert-butoxide, potassium tert-butoxide, tert-butyllithium or n-butyllithium.
(4) The process for producing a 1-pyridin-4-yl-indole as described in any of (1) to (3) above,
wherein the base used is sodium tert-butoxide or potassium tert-butoxide.

The process of the invention permits reduction of industrial production cost by using inexpensively available 4-halopyridines without using heavy metal catalysts. Also, the process of the invention is industrially useful in the points that, in comparison with the heavy metal catalyst-using reaction generally employed, deterioration of product quality due to contamination with a catalyst and environmental pollution due to outflow of heavy metals can be avoided and that works such as removal of catalysts in the step of purifying the product can be eliminated.

### Detailed Description of the Invention

The invention will be described in detail below.

In order to describe the invention in more detail, an example of using sodium tert-butoxide is described below as one example of the process of the invention which, however, do not limit the contents of the invention in any way.

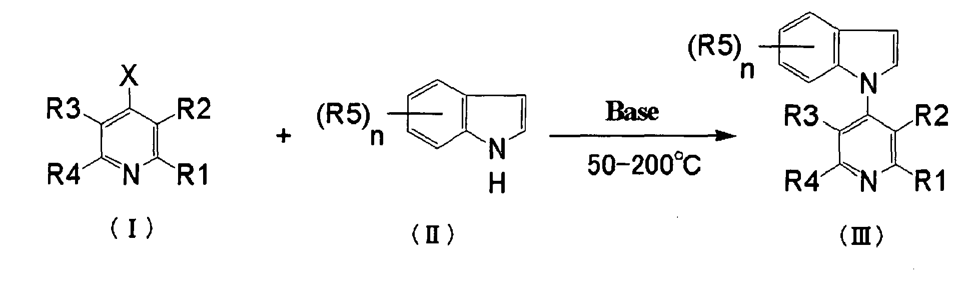

In the above formulae, R1 to R5, X and n are the same as defined hereinbefore.

In the compounds of the invention represented by formula (I), X represents a chlorine atom, a bromine atom or an iodine atom.

In the compounds of the invention represented by formulae (I) to (III), R1 to R5 are not particularly limited as long as they are groups which do not exert detrimental influences on the reaction.

Specifically, R1 and R4 each independently represents a hydrogen atom, an alkyl group, an alkenyl group, an alkynyl group, an aryl group, an alkoxy group, an aryloxy group, an alkylcarbonyloxy group, an arylcarbonyloxy group, a carboxy group, an alkylcarbonyl group, an arylcarbonyl group, an alkoxycarbonyl group, an aryloxycarbonyl group, a sulfo group, an alkylsulfonyl group, an arylsulfonyl group, a carbamoyl group di-substituted by alkyl and/or aryl, a sulfamoyl group di-substituted by alkyl and/or aryl, an alkylthio group, an arylthio group, an alkylthiocarbonyl group, an arylthiocarbonyl group, a nitro group, an amino group di-substituted by alkyl and/or aryl, a cyano group or a hetero ring residue.

R2 and R3 each independently represents a hydrogen atom, an alkyl group, an alkenyl group, an alkynyl group, an aryl group, an alkoxy group, an aryloxy group, an alkylcarbonyloxy group, an arylcarbonyloxy group, a carboxy group, an alkylcarbonyl group, an arylcarbonyl group, an alkoxycarbonyl group, an aryloxycarbonyl group, a sulfo group, an alkylsulfonyl group, an arylsulfonyl group, a carbamoyl group di-substituted by alkyl and/or aryl, a sulfamoyl group di-substituted by alkyl and/or aryl, an alkylthio group, an arylthio group, an alkylthiocarbonyl group, an arylthiocarbonyl group, a nitro group, an amino group di-substituted by alkyl and/or aryl, a cyano group, a chlorine atom, a bromine atom, an iodine atom or a hetero ring residue, with R1 and R2, or R3 and R4, being optionally connected to each other to form a ring.

R5 represents an alkyl group, an alkenyl group, an alkynyl group, an aryl group, an alkoxy group, an aryloxy group, an alkylcarbonyloxy group, an arylcarbonyloxy group, a carboxy group, an alkylcarbonyl group, an arylcarbonyl group, an alkoxycarbonyl group, an aryloxycarbonyl group, a sulfo group, an alkylsulfonyl group, an arylsulfonyl group, a carbamoyl group di-substituted by alkyl and/or aryl, a sulfamoyl group di-substituted by alkyl and/or aryl, an alkylthio group, an arylthio group, an alkylthiocarbonyl group, an arylthiocarbonyl group, a nitro group, an amino group di-substituted by alkyl and/or aryl, a cyano group, a halogen atom or a hetero ring residue.

n represents an integer of from 0 to 6.

The alkyl group represented by R1 to R5 represents a straight, branched or cyclic alkyl group containing from 1 to 20 carbon atoms, such as methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl, cyclopropyl, cyclobutyl, cyclopentyl, cyclohexyl, cycloheptyl, cyclooctyl, cyclononyl or cyclodecyl.

The alkenyl group represented by R1 to R5 represents a straight, branched or cyclic alkenyl group containing from 2 to 20 carbon atoms, such as vinyl, allyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, nonadecenyl, icosenyl, hexadienyl or dodecatrienyl, cyclopentenyl, cyclooctenyl or 1,3-cyclohexadienyl.

The alkynyl group represented by R1 to R5 represents a straight, branched or cyclic alkynyl group containing from 2 to 20 carbon atoms, such as ethynyl, butynyl, pentynyl, hexynyl, heptynyl, octynyl, nonynyl, cyclooctynyl, cyclononynyl or cyclodecynyl.

The aryl group represented by R1 to R5 represents a 5- to 10-membered, monocyclic or bicyclic carbon-containing aryl group such as phenyl or naphthyl.

The alkoxy group represented by R1 to R5 represents an alkoxy group containing from 1 to 20 carbon atoms, such as methoxy, ethoxy, propoxy, butoxy, pentyloxy, hexyloxy, heptyloxy, octyloxy, nonyloxy, decyloxy, dodecyloxy or octadecyloxy.

The aryloxy group represented by R1 to R5 represents, for example, phenoxy or naphthyloxy.

The alkylcarbonyloxy group represented by R1 to R5 represents a carbonyloxy group substituted by an alkyl group containing from 1 to 20 carbon atoms, such as acetoxy, ethylcarbonyloxy, tert-butylcarbonyloxy, n-decylcarbonyloxy, n-hexadecylcarbonyloxy.

The arylcarbonyloxy group represented by R1 to R5 represents a carbonyloxy group substituted by a 5- to 10-membered monocyclic or bi-cyclic aryl group, such as benzoyloxy or naphthylcarbonyloxy.

The carboxy group represented by R1 to R5 includes salts of a free carboxy group such as sodium salt, potassium salt and calcium salt as well as the free carboxy group.

The alkylcarbonyl group represented by R1 to R5 represents a carbonyl group substituted by an alkyl group containing from 1 to 20 carbon atoms, such as acetyl, propionyl, pyvaloyl, butyryl, isobutyryl, valeryl, octanoyl, decanoyl, lauroyl, palmitoyl, stearoyl, cyclobutylcarbonyl, cyclopentylcarbonyl or cyclohexylcarbonyl.

The arylcarbonyl group represented by R1 to R5 represents a carbonyl group substituted by a 5- to 10-membered monocyclic or bi-cyclic aryl group, such as benzoyl or naphthoyl.

The alkoxycarbonyl group represented by R1 to R5 represents a carbonyl group substituted by an alkoxy group containing from 1 to 20 carbon atoms such as methoxycarbonyl, ethoxycarbonyl, tert-butoxycarbonyl, n-hexyloxycarbonyl, n-octyloxycarbonyl, n-decyloxycarbonyl, n-hexadecyloxycarbonyl, cyclopentyloxycarbonyl or cyclohexyloxycarbonyl.

The aryloxycarbonyl group represented by R1 to R5 represents a carbonyl group substituted by an aryloxy group such as phenoxycarbonyl or naphthyloxycarbonyl.

The sulfo group represented by R1 to R5 includes salts of a free sulfo group such as sodium salt, potassium salt and calcium salt as well as the free sulfo group.

The alkylsulfonyl group represented by R1 to R5 represents a sulfonyl group substituted by an alkyl group containing from 1 to 20 carbon atoms, such as methylsulfonyl, ethylsulfonyl, propylsulfonyl, butylsulfonyl, tert-butylsulfonyl, pentylsulfonyl, hexylsulfonyl, octylsulfonyl, dodecylsulfonyl, hexadecylsulfonyl, cyclobutylsulfonyl, cyclopentylsulfonyl or cyclohexylsulfonyl.

The arylsulfonyl group represented by R1 to R5 represents, for example, phenylsulfonyl or naphthylsulfonyl.

The di-substituted carbamoyl group represented by R1 to R5 represents a carbamoyl group di-substituted by an alkyl group containing from 1 to 20 carbon atoms and/or by a 5- to 10-membered monocyclic or bi-cyclic aryl group, such as N,N-dimethylcarbamoyl, N,N-dihexylcarbamoyl, N,N-didodecylcarbamoyl, N-methyl-N-(neo-pentyl)carbamoyl, N,N-diphenylcarbamoyl, N-phenyl-N-methylcarbamoyl or N-naphthyl-N-(tert-butyl)carbamoyl.

The di-substituted sulfamoyl group represented by R1 to R5 represents a sulfamoyl group di-substituted by an alkyl group containing from 1 to 20 carbon atoms and/or by a 5- to 10-membered monocyclic or bi-cyclic aryl group, such as N,N-dimethylsulfamoyl, N,N-diisopropylsulfamoyl, N,N-dioctylsulfamoyl, N,N-ditetradecylsulfamoyl, N-ethyl-N-(tert-butyl)sulfamoyl, N,N-diphenylsulfamoyl or N-ethyl-N-phenylsulfamoyl.

The alkylthio group represented by R1 to R5 represents an alkylthio group containing from 1 to 20 carbon atoms, such as methylthio, ethylthio, isopropylthio, butylthio, neopentylthio, hexylthio, heptylthio, octylthio, nonylthio, decylthio, dodecylthio, hexadecylthio, cyclopentylthio or cyclohexylthio.

The arylthio group represented by R1 to R5 represents, for example, phenylthio or naphthylthio.

The alkylthiocarbonyl group represented by R1 to R5 represents a thiocarbonyl group substituted by an alkyl group containing from 1 to 20 carbon atoms, such as thioacetyl, thiopropionyl, thiobutyryl, thiovaleryl, thiodecanoyl, thiotetradecanoyl, cyclobutylthiocarbonyl or cyclohexylthiocarbonyl.

The arylthiocarbonyl group represented by R1 to R5 represents a thiocarbonyl group substituted by a 5- to 10-membered monocyclic or bi-cyclic aryl group, such as thiobenzoyl or thionaphthoyl.

The di-substituted amino group represented by R1 to R5 represents an amino group di-substituted by an alkyl group containing from 1 to 20 carbon atoms and/or by a 5- to 10-membered monocyclic or bi-cyclic aryl group, such as N,N-diethylamio, N,N-diheptylamino, N,N-dioctylamino, N,N-dodecylamino, N,N-octadecylamino, N-methyl-N-hexylamino, N,N-diphenylamino, N-phenyl-N-naphthylamino or N-ethyl-N-phenylamino.

The halogen atom represented by R5 represents a chlorine atom, a bromine atom, an iodine atom or a fluorine atom.

The hetero ring residue represented by R1 to R5 represents, for example, a 5- to 10-membered monocyclic or bicyclic hetero ring group containing from 1 to 4 atoms selected from among nitrogen, oxygen and sulfur, and specifically represents thienyl, furyl, pyranyl, pyridyl, pyrrolyl, pyrazinyl, azepinyl, azocinyl, azoninyl, azecinyl, oxazolyl, thiazolyl, pyrimidinyl, pyridazinyl, triazinyl, tetrazolyl, imidazolyl, pyrazolyl, morpholinyl, thiomorpholinyl, piperidyl, piperazinyl, quinolyl, isoquinolyl, indolyl, isoindolyl, quinoxalinyl, phthalazinyl, quinolizinyl, quinazolinyl, quinoxalinyl, naphthyridinyl, chromenyl, benzofuryl, benzothienyl or like.

These substituents of R1 to R5 may further have a substituent. Such substituent which these substituents may further have is not particularly limited and is exemplified by an alkyl group, an alkenyl group, an aryl group, an alkoxy group, an aryloxy group, a di-substituted amino group, an alkylthio group, an arylthio group, a halogen atom and a hetero ring residue which, however, are not necessarily limitative. R1 and R2, or R3 and R4, may be connected to each other to form a ring structure. As such ring, partly saturated rings such as cyclopentene, cyclohexene and cyclooctene; aromatic rings such as benzene and naphthalene; and hetero rings such as pyrrole, dihydropyrrole, pyridine, pyran and dihydropyran are illustrated. These rings may further have a substituent which is not particularly limitative.

X is preferably a bromine atom or a chlorine atom, more preferably a chlorine atom.

R1 to R4 are preferably a hydrogen atom, an alkyl group, a nitro group, a cyano group, an alkylcarbonyl group or an arylcarbonyl group, more preferably a hydrogen atom.

R5 is preferably a hydrogen atom, an alkyl group, an aryl group, an alkoxy group, an aryloxy group, an alkylthio group or an arylthio group, more preferably a hydrogen atom.

Specific examples of the compounds represented by formula (III) are shown below. However, the invention is not limited only to these compounds.

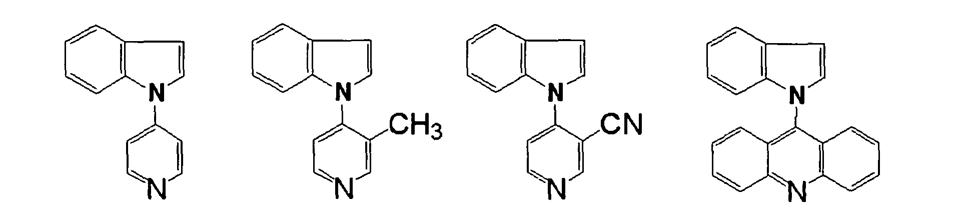

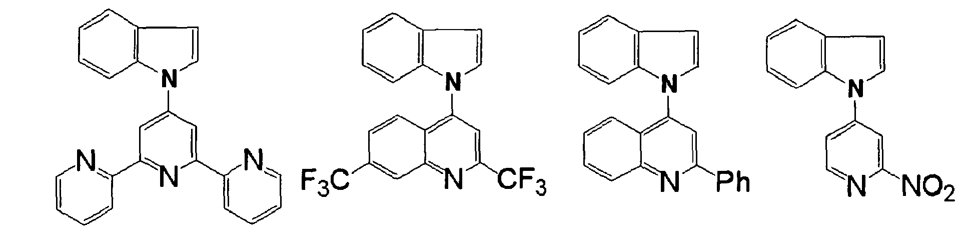

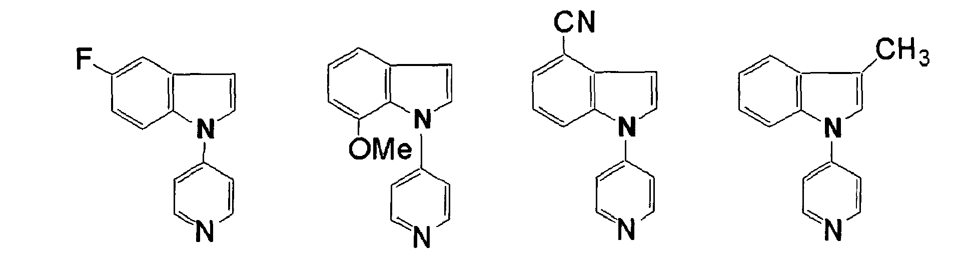

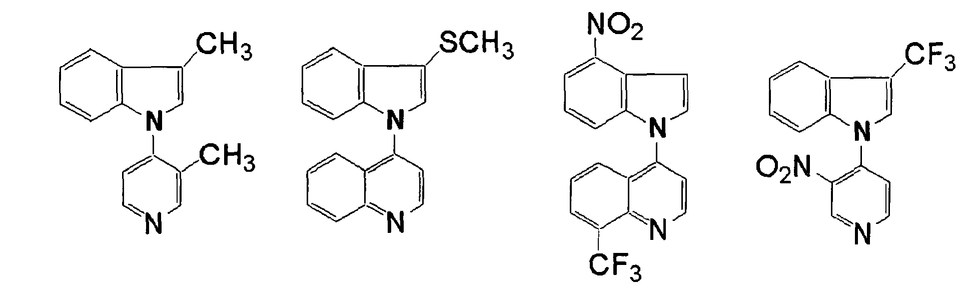

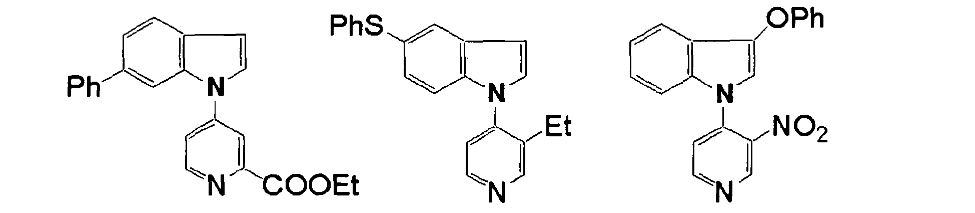

Next, the production process of the invention will be described below.

As the 4-halogenopyridines and the indoles to be used in the invention, various kinds of products are marketed and easily available. The amount of the indoles to be used is from 0.5 to 3 equivalents, preferably from 0.8 to 2 equivalents, more preferably from 0.9 to 1.6 equivalents, based on the 4-halogenopyridines. Additionally, the 4-halogenopyridines of the invention include in their category inorganic salts such as hydrochlorides, sulfates and phosphates and organic acid salts such as acetates, benzoates and methanesulfonates.

The base to be used in the invention can dissociate the NH group of indole to generate an N-anion. The NH-group of indole is usually difficult to dissociate (pKa: 17), and hence a base of 15 or more in pKa in water is preferably used in the invention. Any base that has a pKa of 15 or more in water can be used but, more preferably, inorganic bases such as sodium hydride (pKa: about 35), sodium hydroxide (pKa: 15.7) and potassium hydroxide (pKa: 15.7), alkoxide bases such as sodium methoxide (pKa: 15.5), sodium tert-butoxide (pKa: 17.0) and potassium tert-butoxide (pKa: 17.0) and organometallic bases such as tert-butyllithium (pKa: 53) and n-butyllithium (pKa: 48) are illustrated. Inorganic bases such as sodium hydride are inexpensive, easily available and easily handled. However, in the case where the substrate to be used is particularly a hydrochloride, hydrobromide or sulfate, vigorous foaming occurs due to generation of a hydrogen gas upon charging the substrate, and hence the alkoxide bases which do not provide a danger of explosion are preferred among them in industrializing the process. Of the alkoxide bases, sodium tert-butoxide and potassium tert-butoxide are most preferred. These bases may be used independently or in combination of two or more thereof according to the circumstances.

Additionally, the pKa values described above are values described in MARCH'S Advanced Organic Chemistry, 5th ed., published by WILEY-INTERSCIENCE.

The amount of the base to be used varies depending upon kind of the base to be used and kind of the substituent to be dissociated, but is usually from 0.5 to 5 equivalents, preferably from 0.8 to 4 equivalents, more preferably from 1 to 3 equivalents, based on the 4-halogenopyridines.

The reaction of the invention may be conducted in the absence of a solvent, but a solvent may be used according to the circumstances. As the solvent, those solvents are preferred which have a dielectric constant of from 2 to 50 and, specifically, aromatic solvents such as toluene (dielectric constant: 2.24) and anisole (dielectric constant: 4.33); ether series solvents such as tetrahydrofuran (dielectric constant: 7.58), 1,4-dioxane (dielectric constant: 2.209), ethylene glycol dimethyl ether (dielectric constant: 5.50), ethylene glycol diethyl ether (dielectric constant: 5.10) and diisopropyl ether (dielectric constant: 4.49); amide series solvents such as N,N-dimethylformamide (dielectric constant: 36.71), N,N-dimethylacetamide (dielectric constant: 37.78; hereinafter abbreviated as "DMAc") and 1-methyl-2-pyrrolidinone (dielectric constant: 32.0; hereinafter abbreviated as "NMP"); and nitrile series solvents such as acetonitrile (dielectric constant: 37.5) and propionitrile (dielectric constant: 29.7) are illustrated. These solvents may be used independently or as a mixture of two or more thereof to use. In the case of using as a mixture, the mixing ratio can be determined with no limitation. The amount of the solvent to be used is not particularly limited, but is preferably from 0.1 to 100 times, more preferably from 1 to 50 times, particularly preferably from 2 to 10 times, as much as that of the 4-halogenopyridines.

Additionally, the dielectric constants are values described in Yozai Handbook published by Kodansha in year 1998.

In the invention, additives may be used according to circumstances. As the additives, iodides are illustrated. Specifically, inorganic salts such as potassium iodide and sodium iodide; and quaternary ammonium salts such as tetramethylammonium iodide and tetrabutylammonium iodide are illustrated. It is not clear how these iodides contribute to the reaction, but addition of the additives serves to improve reaction rate, thus being preferred. These iodides can be used independently or as a mixture of two or more thereof and, in the case of using as a mixture, the mixing ratio can be determined with no limitation. The amount of the iodide to be used is not particularly limited, but is preferably from 0.001 to 3 equivalents, more preferably from 0.05 to 1 equivalent, particularly preferably from 0.1 to 0.4 equivalent based on the 4-halogenopyridines.

The reaction temperature of the invention is preferably from 50°C to 200°C, more preferably from 80°C to 180°C, particularly preferably from 100°C to 150°C. In case when the temperature is lower than 50°C, the reaction proceeds extremely slowly whereas, in case when the temperature is higher than 200°C, by-products are produced, thus such temperatures not being preferred.

The reaction time varies depending upon kinds of the substrate and the base to be used, but the reaction is completed usually within 24 hours and, in many cases, in a period of from 1 to 10 hours.

After completion of the reaction, the thus-obtained 1-pyridin-4-yl-indoles can be isolated and purified according to common methods for organic compounds. For example, a crude product can be obtained by adding water to the reaction solution, subjecting it to liquid separation and extraction treatment using a solvent such as ethyl acetate, and concentrating the extract. In the case where purification is required, the crude product is purified by re-crystallization using ethyl acetate, toluene, alcohol or hexane, column purification using silica gel or by distillation under reduced pressure. These purification methods can be employed independently or in combination of two or more thereof to obtain an end product with high purity.

### [Examples]

Next, the invention will be described in more detail by reference to Examples which, however, are not to be construed as limiting the scope of the invention.

### Example 1 Synthesis of 1-pyridin-4-yl-indole

7.81 g (0.067 mol) of indole, 10.00 g (0.067 mol) of 4-chloropyridine hydrochloride and 17.94 g (0.187 mol) of sodium tert-butoxide were added to 80 ml of DMAc, and the mixture was heated to 110°C to react for 2 hours. After completion of the reaction, the reaction mixture was cooled to 25°C, 200 ml of water and 200 ml of ethyl acetate were added thereto and, after liquid-liquid separation, the organic layer was washed with 200 ml of water. The organic layer was concentrated under reduced pressure to obtain 11.66 g (yield: 90%) of the end product as a brown oil.

### Examples 2 to 5, 8 to 12, and 15 to 19

The same procedures as in Example 1 were conducted except for changing the base and the reaction temperature used in Example 1 to those shown in Table 1.

### Example 6 Synthesis of 1-pyridin-4-yl-indole

7.81 g (0.067 mol) of indole, 10.00 g (0.067 mol) of 4-chloropyridine hydrochloride, and 17.94 g (0.187 mol) of sodium tert-butoxide were added to 80 ml of NMP, and the mixture was heated to 180°C to react for 2 hours. After completion of the reaction, the reaction mixture was cooled to 25°C, 200 ml of water and 200 ml of ethyl acetate were added thereto and, after liquid-liquid separation, the organic layer was washed with 200 ml of water. The organic layer was concentrated under reduced pressure to obtain 11.14 g (yield: 86%) of the end product as a brown oil.

### Examples 7, 13, 14, 20, and 21

The same procedures as in Example 6 were conducted except for changing the base and the reaction temperature used in Example 6 to those shown in Table 1. Example 22 Synthesis of 1-pyridin-4-yl-indole

7.81 g (0.067 mol) of indole and 10.00 g (0.067 mol) of 4-chloropyridine hydrochloride were added to 100 ml of 1,4-dioxane, 39.2 ml (1.7 mol/l pentane solution; 0.067 mol) of tert-butyllithium was gradually added dropwise thereto, and the mixture was heated to 110°C to react for 20 hours. After completion of the reaction, the reaction mixture was cooled to 25°C, 200 ml of water and 200 ml of ethyl acetate were added thereto and, after liquid-liquid separation, the organic layer was washed with 200 ml of water. The organic layer was concentrated under reduced pressure to obtain 9.33 g (yield: 72%) of the end product as a brown oil.

### Comparative Examples 1 to 6

The same procedures as in Example 1 were conducted except for changing the reaction temperature and the base employed in Example 1 to those shown in Table 1.

Results of Examples 1 to 22 and Comparative Examples 1 to 6 are shown in Table 1.

**Table 1**

| | Base | Reaction Solvent | Reaction Temperature (°C) | Yield (%) |
|---|---|---|---|---|
| Example 1 | tert-BuONa | DMAc | 110 | 90 |
| Example 2 | tert-BuONa | DMAc | 50 | 12 |
| Example 3 | tert-BuONa | DMAc | 60 | 21 |
| Example 4 | tert-BuONa | DMAc | 80 | 59 |
| Example 5 | tert-BuONa | DMAc | 150 | 93 |
| Example 6 | tert-BuONa | NMP | 180 | 86 |
| Example 7 | tert-BuONa | NMP | 200 | 74 |
| Example 8 | tert-BuOK | DMAc | 50 | 11 |
| Example 9 | tert-BuOK | DMAc | 60 | 20 |
| Example 10 | tert-BuOK | DMAc | 80 | 40 |
| Example 11 | tert-BuOK | DMAc | 110 | 80 |
| Example 12 | tert-BuOK | DMAc | 150 | 92 |
| Example 13 | tert-BuOK | NMP | 180 | 82 |
| Example 14 | tert-BuOK | NMP | 200 | 75 |
| Example 15 | NaH | DMAc | 50 | 14 |
| Example 16 | NaH | DMAc | 60 | 22 |
| Example 17 | NaH | DMAc | 80 | 75 |
| Example 18 | NaH | DMAc | 110 | 92 |
| Example 19 | NaH | DMAc | 150 | 93 |
| Example 20 | NaH | NMP | 180 | 88 |
| Example 21 | NaH | NMP | 200 | 71 |
| Example 22 | tert-BuLi | 1,4-dioxane | 110 | 72 |
| Comparative Example 1 | tert-BuONa | DMAc | 25 | 1 |
| Comparative Example 2 | tert-BuONa | DMAc | 40 | 4 |
| Comparative Example 3 | tert-BuOK | DMAc | 25 | 1 |
| Comparative Example 4 | tert-BuOK | DMAc | 40 | 3 |
| Comparative Example 5 | NaH | DMAc | 25 | 1 |
| Comparative Example 6 | NaH | DMAc | 40 | 4 |

As is apparent from the results shown in Table 1, the end product can be obtained in good yield according to the invention even when 4-chloropyridine, which has not conventionally caused the reaction, was used as a substrate. On the other hand, when the reaction temperature was low (25°C and 40°C), the reaction scarcely proceeds.

### Example 23 Synthesis of 1-pyridin-4-yl-indole

7.81 g (0.067 mol) of indole, 10.00 g (0.067 mol) of 4-chloropyridine hydrochloride, 2.21 g (0.013 mol) of potassium iodide and 17.94 g (0.187 mol) of sodium tert-butoxide were added to 80 ml of DMAc, and the mixture was heated to 110°C to react for 2 hours. After completion of the reaction, the reaction mixture was cooled to 25°C, 200 ml of water and 200 ml of ethyl acetate were added thereto and, after liquid-liquid separation, the organic layer was washed with 200 ml of water. The organic layer was concentrated under reduced pressure to obtain 12.18 g (yield: 94%) of the end product as a brown oil.

### Examples 24 and 25

The same procedures as in Example 23 were conducted except for changing the base employed in Example 23 to those shown in Table 2.

Results of Examples 23 to 25 are shown in Table 2.

**Table 2**

| | Addition of KI | Base | Yield (%) |
|---|---|---|---|
| Example 23 | yes | tert-BuONa | 94 |
| Example 24 | yes | tert-BuOK | 86 |
| Example 25 | yes | NaH | 94 |
| Example 1 | no | tert-BuONa | 90 |
| Example 11 | no | tert-BuOK | 80 |
| Example 18 | no | NaH | 92 |

### Example 26 Synthesis of 1-pyridin-4-yl-indole

7.81 g (0.067 mol) of indole, 12.96 g (0.067 mol) of 4-bromopyridine hydrochloride, and 17.94 g (0.187 mol) of sodium tert-butoxide were added to 80 ml of DMAc, and the mixture was heated to 110°C to react for 4 hours. After completion of the reaction, the reaction mixture was cooled to 25°C, 200 ml of water and 200 ml of ethyl acetate were added thereto and, after liquid-liquid separation, the organic layer was washed with 200 ml of water. The organic layer was concentrated under reduced pressure to obtain 11.40 g (yield: 88%) of the end product as a brown oil.

### Example 27 Synthesis of 1-pyridin-4-yl-indole

7.81 g (0.067 mol) of indole, 16.10 g (0.067 mol) of 4-iodopyridine hydrochloride, and 17.94 g (0.187 mol) of sodium tert-butoxide were added to 80 ml of DMAc, and the mixture was heated to 110°C to react for 10 hours. After completion of the reaction, the reaction mixture was cooled to 25°C, 200 ml of water and 200 ml of ethyl acetate were added thereto and, after liquid-liquid separation, the organic layer was washed with 200 ml of water. The organic layer was concentrated under reduced pressure to obtain 9.72 g (yield: 75%) of the end product as a brown oil.

### Examples 28 to 32

The same procedures as in Example 1 were conducted except for changing the substrate employed in Example 1 to those shown in Table 3.

Results are shown in Table 3.

**Table 3**

| **Example** | **Pyridine Compound** | **Indole Compound** | **End Product** | **Yield(%)** |
|---|---|---|---|---|
| 28 | 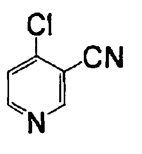 | 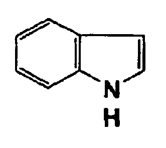 | 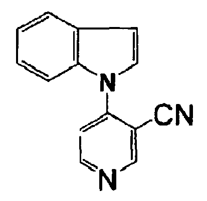 | 92 |
| 29 | 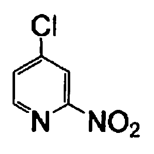 | 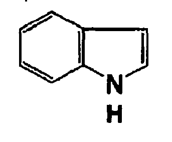 | 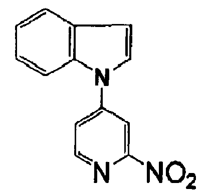 | 94 |
| 30 | 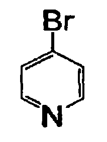 | 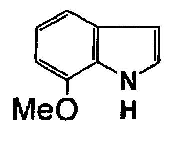 | 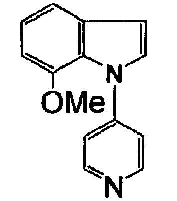 | 90 |
| 31 | 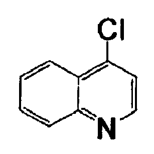 | 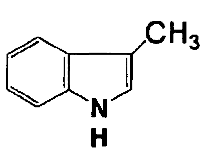 | 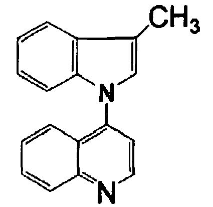 | 92 |
| 32 | 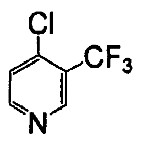 | 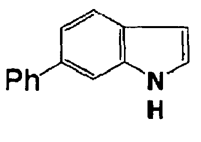 | 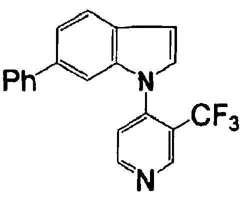 | 89 |

The invention enables production of 1-pyridin-4-yl-indoles useful in the fields of medicines, agricultural chemicals, organic electroluminescence elements, catalyst ligands and solar cell elements in high yield on an industrial scale inexpensively.

The entire disclosure of each and every foreign patent application from which the benefit of foreign priority has been claimed in the present application is incorporated herein by reference, as if fully set forth.

## Claims

1. A process for producing a 1-pyridin-4-yl-indole represented by formula (III), which comprises reacting a pyridine compound represented by formula (I) with an indole compound represented by formula (II) in the presence of a base at 50 to 200°C: 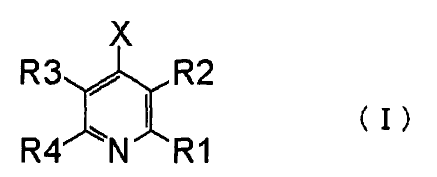 wherein X represents a chlorine atom, a bromine atom or an iodine atom;
R1 and R4 each independently represents a hydrogen atom, an alkyl group, an alkenyl group, an alkynyl group, an aryl group, an alkoxy group, an aryloxy group, an alkylcarbonyloxy group, an arylcarbonyloxy group, a carboxy group, an alkylcarbonyl group, an arylcarbonyl group, an alkoxycarbonyl group, an aryloxycarbonyl group, a sulfo group, an alkylsulfonyl group, an arylsulfonyl group, a carbamoyl group di-substituted by at least one of alkyl and aryl, a sulfamoyl group di-substituted by at least one of alkyl and aryl, an alkylthio group, an arylthio group, an alkylthiocarbonyl group, an arylthiocarbonyl group, a nitro group, an amino group di-substituted by at least one of alkyl and aryl, a cyano group or a hetero ring residue;
R2 and R3 each independently represents a hydrogen atom, an alkyl group, an alkenyl group, an alkynyl group, an aryl group, an alkoxy group, an aryloxy group, an alkylcarbonyloxy group, an arylcarbonyloxy group, a carboxy group, an alkylcarbonyl group, an arylcarbonyl group, an alkoxycarbonyl group, an aryloxycarbonyl group, a sulfo group, an alkylsulfonyl group, an arylsulfonyl group, a carbamoyl group di-substituted by at least one of alkyl and aryl, a sulfamoyl group di-substituted by at least one of alkyl and aryl, an alkylthio group, an arylthio group, an alkylthiocarbonyl group, an arylthiocarbonyl group, a nitro group, an amino group di-substituted by at least one of alkyl and aryl, a cyano group, a chlorine atom, a bromine atom, an iodine atom or a hetero ring residue, with R1 and R2, or R3 and R4, being optionally connected to each other to form a ring; 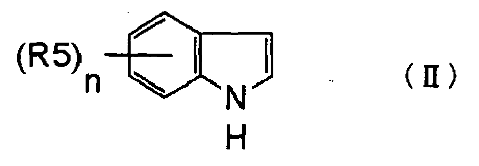 wherein R5 represents an alkyl group, an alkenyl group, an alkynyl group, an aryl group, an alkoxy group, an aryloxy group, an alkylcarbonyloxy group, an arylcarbonyloxy group, a carboxy group, an alkylcarbonyl group, an arylcarbonyl group, an alkoxycarbonyl group, an aryloxycarbonyl group, a sulfo group, an alkylsulfonyl group, an arylsulfonyl group, a carbamoyl group di-substituted by at least one of alkyl and aryl, a sulfamoyl group di-substituted by at least one of alkyl and aryl, an alkylthio group, an arylthio group, an alkylthiocarbonyl group, an arylthiocarbonyl group, a nitro group, an amino group di-substituted by at least one of alkyl and aryl, a cyano group, a halogen atom or a hetero ring residue;
n represents an integer of from 0 to 6, and when n represents 2 or more, R5's may be the same or different from each other; and 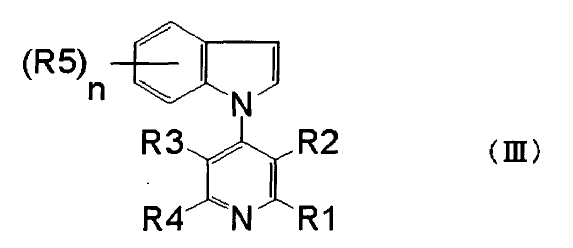 wherein R1 to R5 and n are the same as defined above.

2. The process for producing a 1-pyridin-4-yl-indole according to claim 1,
wherein pKa of the base used is 15 or more.

3. The process for producing a 1-pyridin-4-yl-indole according to claim 1,
wherein the base used is sodium hydride, sodium hydroxide, potassium hydroxide, sodium methoxide, sodium tert-butoxide, potassium tert-butoxide, tert-butyllithium or n-butyllithium.

4. The process for producing a 1-pyridin-4-yl-indole according to claim 1,
wherein the base used is sodium tert-butoxide or potassium tert-butoxide.
